# EUROPEAN PATENT APPLICATION

(11) **EP 4 534 069 A2**
(43) Date of publication of application: **09.04.2025**
(21) Application number: 24203087.2
(22) Date of filing: 27.09.2024
(51) Int. Cl.: A61K 8/22, A61Q 5/10

(54) **A NEW GENERATION HAIR DYEING KIT AND APPLICATION METHOD FOR HOME USE**

(30) Priority: 03.10.2023 TR 202312389
(71) Applicant: Lila Kozmetik Sanayi Anonim Sirketi, 21010 Diyarbakir (TR)
(72) Inventor: CANSIZ, MEHMET, 21010 Diyarbak r (TR)
(74) Representative: Yamankaradeniz, Kemal

(57) **Abstract**

The invention relates to a new generation hair dyeing kit and application method for use at home. The kit, sold as the Sea Color Home Colorist Series, has a formula containing effective color pigments that penetrate from root to tip for rich, luxurious colors. Permanent cream hair dye gives intense color to the hair with its excellent white coverage feature. With its special content, it increases the long-term durability of hair color by trapping more dye in the hair. The inventive hair dyeing kit enables the user to perform processes such as more intense white coverage, increasing colour intensity and preventing unwanted redness at home without the need for professional channels.

## Description

### Field of the Invention

The invention relates to a new generation hair dyeing kit and application method for use at home. The kit, sold as the Sea Color Home Colorist Series, has a formula containing effective color pigments that penetrate from root to tip for rich, luxurious colors. Permanent cream hair dye gives intense color to the hair with its excellent white coverage feature. With its special content, it increases the long-term durability of hair color by trapping more dye in the hair.

### State of the art

Dissatisfaction with hair color has led men and women to seek solutions to change hair color since ancient times. The first records of hair coloring date back to the traditions of Ancient Egypt and China, as well as the contemporary period in the Indian subcontinent. Ancient civilizations derived coloring substances from materials provided by nature. For this, they used fruits, leaves, flowers, roots and tree bark, as well as various minerals and biological resources. The method widely used as today's hair colorant was developed at the end of the 19th century. This method is based on oxidation of precursors and binders. In other words, oxygen is provided by oxidizers containing diluted hydrogen peroxide, and after being applied to the hair and left for 30-40 minutes, the colorants can give the desired color to the hair. This method continues to be used today, largely unchanged.

Nowadays, hair dyeing/coloring can be applied in hairdressing salons or at home. Consumers can have the opportunity to dye their hair at home with the set of dyes that contain all the ingredients needed for hair dyeing, purchased from the markets. In recent years, rapidly increasing salon fees have led consumers to dye their hair at home. Dyeing hair at home has some disadvantages compared to hair dyeing in hair salons. In salon applications, professionals perform the dyeing process by mixing different dyes depending on the customer's hair colour and white intensity in order to achieve better white coverage, prevent the formation of unwanted tones in the hair and obtain more intense colours. With this technique, they obtain more accurate and healthier results. Consumers who dye at home sometimes do not achieve very good results as much as the hairdresser service because they do not know or cannot apply these techniques, and they often encounter problems such as not covering their whites well and unwanted redness on the hair.

The patent application with publication numbered TR2022/019007 relates to the hair dye mixture used to dye people's hair. The invention relates to the hair dye mixture used to dye/coloring people's hair. The invention is particularly related to a hair dye mixture containing lemon salt, rock salt and vinegar, which is a colorant that gives color to the hair, can give color to the hair quickly, increases the permanence of hair color and is used to strengthen the hair.

The European patent with publication numbered EP1555993B1 describes compositions and methods for obtaining permanent color change in hair. These compositions comprise at least one oxidation-based dye precursor for hair; a metal cyanate and an alkalizing agent and an oxidizing compound.

As a result due to the abovementioned disadvantages and the insufficiency of the current solutions regarding the subject matter, a development is required to be made in the relevant technical field.

### Object of the Invention

The present invention aims to solve the abovementioned disadvantages by being inspired from the current conditions.

The object of the invention is to enable the user to perform processes such as more intense white coverage, increasing colour intensity and preventing unwanted redness at home without the need for professional channels.

In order to fulfill the abovementioned aims, the invention is a new generation hair coloring kit developed for home use;
- At least one tube of cream hair dye comprising the first coloring phase containing hair dye pigments;
- At least one tube of oxidant cream that activates the coloring phase characterized by comprising the following;
- At least one tube of assistant cream hair dye comprising the coloring second phase consisting of hair dye pigments that help to obtain (it can be said) the desired color.

The application method of the hair dyeing kit mentioned in any of the above claims is within the scope of the invention, comprises the following process steps:
- mixing the tube of cream hair dye comprising the first colorant phase and the tube assistant hair dye comprising the second colorant phase;
- After obtaining a homogeneous mixture, applying the prepared mixture to the hair;
- Leaving the mixture on the hair for a waiting period of 20-45 minutes,
- Washing dyed hair.

The structural and characteristic features and all advantages of the invention will be more clearly understood with the detailed description given below and therefore the evaluation should be made by taking into account the detailed description.

### Detailed Description of the Invention

In this detailed description, the preferred embodiments of the inventive hair dyeing kit are described by means of examples only for clarifying the subject matter.

With Sea Color Home Colorist Series, it is aimed that the user can perform processes such as more intense white coverage, increasing colour intensity and preventing unwanted redness formation at home without the need for professional channels.

With the support of the assistant dyes in Sea Color Home Colorist Set Dye, it becomes possible for the consumer to achieve the desired hair color without going to hairdressers. With this product, the aim is for the consumer to achieve healthy results at home by using the techniques applied in hair salons.

A 30 ml tube of dye, called assistant dye, has been added to the dye set to support the main color.
**Assistant dye support also aims to provide the following;
   - Better white coverage,
   - More vibrant color results,
   - Neutralizes unwanted redness that may occur with some colors.

An example of the kit according to the invention comprises the following.
- 2 Tubes Cream Hair Dye (50 ml)
- 1 Tube Assistant Cream Hair Dye (30 ml)
- 2 Oxidant Cream (2x65 ml)
- 1 Hair Care Cream (10 ml)
- 1 Pair of Gloves

Tube Cream Hair Dye consists of a coloring phase containing hair dye pigments. Tube assistant cream hair dye consists of a coloring second phase that helps achieve the desired color. Oxidant cream provides activation of the coloring phase. Hair care cream is the care cream used after hair dyeing. Pair of gloves are used to prevent hair dye from getting on hands.

The single color option in a single box, which is among the set dyes available in the market as a grocery product, narrows the personal scales for users. However, thanks to the developed assistant dye, it is aimed to provide users with a more specific and person-based ease of use. In this way, users can adjust the preferred dye amount and color intensity according to their own hair type.

The instructions for use of the hair dyeing kit subject to the invention are given below:
CHOICE: FOR WHITE HAIR
Mixing Ratio: 1 Tube of Cream Dye (50 ml) + 1 Oxidant Cream (65 ml) + ½ Tube of Assistant Dye (15 ml)
PREPARATION:
   - Wear gloves.
   - Squeeze 1 tube of cream hair dye (50 ml) and assistant dye (15 ml) into the oxidant cream bottle. (For each 50 ml tube of hair dye, use 15 ml of 30 ml of assistant dye up to the marked line).

You can preferably prepare the dye mixture in a hair dye bowl or a suitable non-metal container and apply it with a paint brush.
- Close the cap of the oxidant cream bottle and shake well until completely mixed.
- Immediately cut off the tip of the oxidant cream bottle or open the cap slowly to release the gas released during mixing.

### APPLICATION:

- Divide your hair in half. Start the dyeing process from the roots of your hair. Leave for 25 minutes.
- Then divide the hair into small strands and apply the remaining dye mixture to the lengths and ends of your hair. Massage your hair to distribute the dye evenly. Leave for 20 minutes.

The mixture may change color during application. This change has no effect on the color result. Also use the entire dye mixture. Do not store the mixture as it will lose its effect after a certain period of time.
- Wash your hair with warm water and lather it. Then rinse thoroughly until the water runs clear.
- Gently spread the hair care cream on the lengths and ends of your wet hair and massage. Rinse your hair after waiting for 2 minutes.

### II. CHOICE: FOR MORE INTENSIVE AND VIBRANT COLORS

Mixing Ratio: 2 Tubes of Cream Dye (2x50ml) + 2 Tubes of Oxidant Cream (2×65ml) + 1 Tube of Assistant Dye (30 ml)

### PREPARATION:

Wear gloves.

Prepare 2 tubes of cream hair dye, 1 tube of assistant dye and 2 tubes of oxidant cream in a dye bowl or a suitable non-metallic container and apply with a paint brush.

### APPLICATION:

- Divide your hair in half. Start the dyeing process from the roots of the hair, then divide the hair into small strands and dye from roots to ends.
- Apply the dye applied to the entire hair by massaging it to distribute it evenly, then leave it for 45 minutes.

The mixture may change color during application. This change has no effect on the color result. Also use the entire dye mixture. Do not store the mixture as it will lose its effect after a certain period of time.
- Wash your hair with warm water and lather it. Then rinse your hair until the water becomes clear.
- Gently spread the hair care cream on the lengths and ends of your wet hair and massage. Rinse your hair after waiting for 2 minutes.

## Claims

1. A new generation hair dyeing kit, which is developed for use at home
• at least one tube of cream hair dye comprising the first coloring phase containing hair dye pigments
• at least one tube of oxidant cream that activates the coloring phase
***characterized by** comprising;*
• at least one tube of assistant cream hair dye comprising the second coloring phase consisting of hair dye pigments that help to obtain the desired color.

2. The kit according to Claim 1, **characterized by** comprising; at least one tube of hair care cream used after the hair dye process.

3. The kit according to Claim 1, **characterized by** comprising; at least one pair of gloves used to prevent hair dye from contaminating hands.

4. The kit according to Claim 1 to 4, **characterized by** comprising the following:
• At least two tubes of 50 ml cream hair dye
• At least one tube of 30 ml assistant hair dye cream
• At least two tubes of 65 ml oxidant cream
• At least one tube of 10ml hair care conditioner
• at least a pair of gloves.

5. An application method of the hair dyeing kit according to any of the preceding claims, **characterized by** comprising the following process steps:
• mixing the tube of cream hair dye comprising the first colorant phase and the tube assistant hair dye comprising the second colorant phase;
• after obtaining a homogeneous mixture, applying the prepared mixture to the hair;
• Leaving the mixture on the hair for a waiting period of 20-45 minutes,
• washing dyed hair.

6. The method according to claim 5, **characterized by** comprising of the following process steps; for complete dyeing of white hair;
• preparing one tube of cream dye (50 ml), 1 tube of oxidant cream (65 ml) and half a tube of assistant dye (15 ml);
• preferably wearing gloves;
• use 15 ml of 30 ml of assistant dye for each 50 ml of hair dye;
• squeeze tube cream hair dye (50 ml) and assistant dye (15 ml) into the oxidant cream tube;
• closing the cap of the oxidant cream bottle and shaking well until completely mixed;
• immediately cutting off the tip of the oxidant cream bottle or slowly opening the lid to expel the gas released during mixing;
• parting your hair in half; starting the dyeing process from the roots of the hair and waiting for 25 minutes;
• then dividing the hair into small strands and apply the remaining dye mixture to the lengths and ends of the hair; massaging the hair and leaving it for 20 minutes to distribute the dye evenly;
• washing hair with warm water; rinsing thoroughly until the water runs clear;
• gently spreading the hair care cream on the lengths and ends of your wet hair, massaging it and rinsing your hair after waiting for 2 minutes.

7. The method according to Claim 5, **characterized by** comprising the following process steps for more intense and vibrant colors;
• preparing two tubes of cream dye (2x50 ml), two tubes of oxidant cream (2x65 ml) and one tube of assistant dye (30 ml);
• preferably wearing gloves;
• mixing two tubes of cream hair dye, one tube of assistant dye, two tubes of oxidant cream in a bowl;
• splitting your hair in half; starting the dyeing process from the roots of the hair, then dividing the hair into small strands and dyeing it from roots to ends;
• massaging the hair and leaving it for 45 minutes to ensure that all the dye is distributed evenly;
• washing hair with warm water; rinsing thoroughly until the water runs clear;
• gently spreading the hair care cream on the lengths and ends of your wet hair, massaging it and rinsing your hair after waiting for 2 minutes.
